# EUROPEAN PATENT APPLICATION

(11) **EP 2 182 361 A1**
(43) Date of publication of application: **05.05.2010**
(21) Application number: 09008369.2
(22) Date of filing: 26.06.2009
(51) Int. Cl.: G01N 35/00, G01N 25/04, G01N 33/04

(54) **Device for the detection of the cryoscopic point with multiple and independant measurements**

(30) Priority: 27.06.2008 IT BO20080409
(71) Applicant: Scarpellini, Alba, 50031 Barberino del Mugello, FI (IT); Mugnai, Giancarlo, 50031 Barberino del Mugello, FI (IT)
(72) Inventor: Scarpellini, Alba, 50031 Barberino del Mugello, FI (IT); Mugnai, Giancarlo, 50031 Barberino del Mugello, FI (IT)
(74) Representative: Paolini, Elena

(57) **Abstract**

The device consists of independent measuring probes (8), in number from two as requesting each time, with for each measuring probe a thermistor cryoscope (9) independent to the others, an analogic/digital conversion device (10) for each cryoscope with small diameter temperature probe (11) and a movement apparatus (12) independent for each measuring probe (8).

## Description

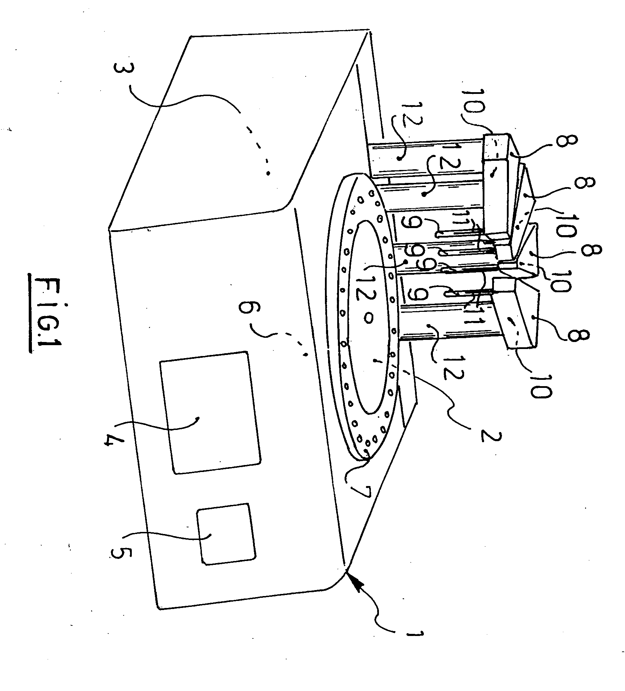

In the field of the devices to verify the presence or less of water in the milk, and consequently in its by-products, thermistor cryoscopes are used. These devices are able to analyse the freezing point of the milk basing their analysis on the fact that the presence of water in the examined sample brings the freezing point nearest to the temperature of 0°C. The milk, collected from the stalls of the producing farms, is put into a tanker with said tanker that can have different compartments so that, in each compartment, is collected the milk of a single producing farm. Putting the collecting milk into a separate compartment for each producing farm is necessary condition to state the milk to be examined traceability so to can identify from where coming the eventual adding of water. To improve the speed of the analysis of the samples coming from the different producing farms are used multi-sample carrying plates with single measuring probe equipped with a thermistor cryoscope. In the art are known also thermistor cryoscopes with double measurement probe placed in parallel and double disposition of sample carrying plates to cut the analysis times in half. The known realizations do not, however, resolve the problem to analyse in real time samples coming from the different producing farms in the same time, so as contemporary to analyze milks of different kinds. The different kinds of milk have, in fact, a different freezing point. Subject-matter of the present invention is a measuring device of the milk freezing point having independent thermistor cryoscopes. Having more thermistor cryoscopes independent each other it is possible to have measurements obtained in quicker way in comparison with the devices known in the art. Moreover, it is possible to probe onto a same sample carrying plates measurements of the freezing point of milk coming to different producing farms, retaining the traceability of the examined milk, and it is possible to actuate the contemporary analysis of different kinds of milks employed the same time of a single analysis. The quality control of different samples, to be arranged contemporary by means of the subject-matter of the invention, determines an advantage not only for the analysis but also in the entire process of production. In the working and in the making up of the product, in fact, the control arranged with the invention permits to avoid wait times in presence of samples to be examined of different kinds. So, the analysis speed of the different samples to be examined permits to begin in advance the production process to produce the different by-products from the collected milk like fresh milk, long and short conservation milk, cream, cheese, ricotta, butter, yogurt or other by-product based on caw, sheep, goat or buffalo milk. It is so obtained the final product to be commercialized from the specific raw material in short time, with obviously advantages for the products in which the time necessary to pass from the raw material to the final product to be commercialized is of primary importance to guarantee the freshness. Moreover, the invented device is equipped with a analogic/digital conversion device directly onto each measuring probe, so to have the signal coming to the measuring probe of each single thermistor cryoscope that is immediately converted in digital onto the same measuring probe. This permits to the subsequent signal transfer inside the invented device to be noise-free that could falsify the precision of the same measurement. The potential differences used in the thermistor cryoscope are, in fact, in the order with less value than a millesimal of Volt and also small interferences or noises can falsify the measuring of the freezing point. With the analogic/digital conversion device directly on each measuring probe, the invention provides, moreover, measuring probes with less diameter than those currently used to have reduced thermic inertia. Moreover, having the invented device movement apparatuses of the samples to be examined independent for each single measuring probe with thermistor cryoscope, as soon as ended the measuring of the freezing point, the single probe gets up again, permitting to the operator to actuate possible cleaning operations of the probe or to remove the sample without must to wait the ending of the other measurements. The invented device is illustrated in a merely indicative way in the drawing of sheet 1 - figure 1. Said figure 1 is perspective view of a device with four independent measuring probes. It is to be considered that the invented device may have from two independent measuring probes with consequent apparatuses as independent measuring probes with thermistor cryoscope are requested for the analysis necessities with necessary movement apparatuses of the samples and connected apparatuses. In a preferred embodiment but not exclusive the invented device for the search of the cryoscopic point with multiple and independent measurements consists of, like the conventional devices known in the art, an apparatus 1 with inside cooling tanks 2, electronic devices 3, a control display 4, a printer to print the analysis results 5 and moving means 6 of the carrying sample plate 7. New characteristic of the invented device is to provide independent measuring probes 8 with, for each measuring probe, a thermistor cryoscope 9 independent to the others, an analogic/digital conversion device 10 for each cryoscope with small diameter temperature probes 11, i.e. with diameter less than a millimetre, and movement apparatus 12 independent for each measuring probe 8. The used components and the connection of the different components are of conventional realization following the actuation on the base of the invented device with measuring probes 8 and connected apparatuses independent each others. In working phase the invented device provides the placing of test-tube in the carrying sample plate 7 then put in motion by the moving means 6. Then, each measuring probe 8 provides to arrange the proper analysis of the sample of milk contained in the tube to be analysed in independent way to the other measuring probe 8 by the corresponding thermistor cryoscope 9, the analogic/digital conversion device 10 and the small diameter temperature probe 11, with independent movement apparatus 12 which determines the separated movement of each measuring probe 8. Actuated the analysis and with the data for each sample printed and/or displayed onto the control display 4, each measuring probe 8 moves automatically and independently to the others and it prepares itself to analyze an other milk sample both coming to a different producing farm than to a different kind of milk. The invented device is possible of a lot of modifications and changings on the base of the different use necessities all parts of the present subject-matter. Moreover, all the technical parts are to be changed with other technical equivalent.

## Claims

1. Device for the detection of the cryoscopic point with multiple and independent measurements comprising an apparatus (1) with inside cooling tanks (2), electronic devices (3), a control display (4), a printer to print the analysis results (5) and moving means (6) of the carrying sample plate (7); **characterized in that** to have independent measuring probes (8) with, for each measuring probe, a thermistor cryoscope (9) independent to the others, an analogic/digital conversion device (10) for each cryoscope with small diameter temperature probe (11), i.e. with diameter less than a millimetre, and movement apparatus (12) independent for each measuring probe (8).

2. Device for the detection of the cryoscopic point with multiple and independent measurements, as per claim 1, **characterized in that** it can have from two independent measuring probes with consequent apparatuses as independent measuring probes with thermistor cryoscope are requested for the analysis necessities, with necessary movement apparatuses of the samples and connected apparatuses.
